(19)

Europäisches
Patentamt
European
Patent Office
Office européen
des brevets

(11) **EP 4 523 619 A1**

(12) **EUROPEAN PATENT APPLICATION**
published in accordance with Art. 153(4) EPC

(43) Date of publication:
**19.03.2025 Bulletin 2025/12**

(21) Application number: **23803558.8**

(22) Date of filing: **09.05.2023**

(51) International Patent Classification (IPC):
**A61B 5/08** (2006.01)          **A61B 5/00** (2006.01)

(52) Cooperative Patent Classification (CPC):
**A61B 5/00; A61B 5/08**

(86) International application number:
**PCT/JP2023/017426**

(87) International publication number:
**WO 2023/219081 (16.11.2023 Gazette 2023/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.05.2022   JP 2022077445**

(71) Applicants:
• **UNIVERSITY PUBLIC CORPORATION OSAKA**
  **Osaka-shi**
  **Osaka 5998531 (JP)**

• **Iida Sangyo Co., Ltd.**
  **Musashino-shi, Tokyo 180-0022 (JP)**

(72) Inventors:
• **SUZUKI, Takashi**
  **Osaka-shi, Osaka 558-8585 (JP)**
• **MORI, Kazuhiko**
  **Musashino-shi, Tokyo 180-0022 (JP)**
• **MATSUMOTO, Koichi**
  **Musashino-shi, Tokyo 180-0022 (JP)**

(74) Representative: **Mewburn Ellis LLP**
  **Aurora Building**
  **Counterslip**
  **Bristol BS1 6BX (GB)**

(54) **BREATH VISUALIZATION SYSTEM AND METHOD, AND BREATH EVALUATION SYSTEM AND METHOD**

(57)     Provided are a breath visualization system and method, a breath evaluation system and method, and a health evaluation system and method having simple configurations and capable of ensuring visualization of breath even when a front image of a face of a subject is captured by a camera. A breath visualization system comprising: an infrared camera that detects light in the infrared region emitted from a face of a subject to obtain image data; a processing unit that subtract, from first image data obtained at a first time (certain arbitrarily selected time), second image data obtained at second time (prior to the certain arbitrarily selected time) prior to the first time, to generate processed image data; and an output unit that outputs the processed image data.

FIG. 3

```
                    ┌─────────┐
                    │  Start  │
                    └────┬────┘
                         │ ◄─────────┐
      ┌──────────────────┴──────────────────┐
S1 ───┤ Capture face of subject by infrared │
      │ camera                              │
      └──────────────────┬──────────────────┘
                         │
      ┌──────────────────┴──────────────────┐
S2 ───┤ Subtract average of past image data │
      │ from current image data             │
      └──────────────────┬──────────────────┘
                         │
      ┌──────────────────┴──────────────────┐
S3 ───┤      Output processed image data     │
      └──────────────────┬──────────────────┘
                         │
                         └─────────────┘
```

EP 4 523 619 A1

**Description**

TECHNICAL FIELD

The present invention relates to a breath visualization system and method, a breath evaluation system and method, and furthermore to a health evaluation system and method.

BACKGRAUND ART

Biological signals such as body temperature, respiration, and heart rate variability are important parameters for understanding human health. The appearance of wearable devices represented by smart watches has made the measurement of these biological signals easier.

However, if these biological signals could be measured in a non-contact manner, it is possible to measure continuously without causing stress to the subject. For example, as to biosensing by using camera images, it is expected to be applied to sensor-less monitoring at the bedside and remote medical care.

For example, Non-Patent Document 1 discloses non-contact measurement of heart rate and respiration from face images captured by a CMOS camera and a thermography camera. However, the disclosed measurement of Non-Patent Document 1 uses two or more cameras, which complicates the system and increases manufacturing costs.

In Non-Patent Document 2, visualization of respiration rate is attempted by combining an infrared camera and a bandpass filter in the $CO_2$ absorption band. However, in the disclosure of Non-Patent Document 2, the subject needs to turn sideways to the camera in order to distinguish the exhaled breath from the background, which significantly limits the situations where being able to use.

PRIOR ART REFERENCE

Non-Patent Reference

Non-Patent Document 1: G. Sun et al, "Remote sensing of multiple vital signs using a CMOS camera-equipped infrared thermography system and its clinical application in rapidly screening patients with suspected infectious diseases", International Journal of Infectious Diseases 55 (2017) 113-117

Non-Patent Document 2: J. Fei et al, "Imaging Breathing Rate in the CO2 Absorption Band", Proceedings of the 2005 IEEE 700-705

SUMMARY OF THE INVENTION

Problem to be solved by the invention

One of the objects of the present invention is to provide a exhaled breath visualization system and method having simple configurations and capable of ensuring visualization of exhaled breath even when a front image of a face of a subject is captured by a camera. Another object of the present invention is to provide a highly convenient exhaled breath evaluation system and method by utilizing the above-mentioned exhaled breath visualization. Another object of the present invention is further to provide a health evaluation system and method which includes the exhaled breath evaluation system and method.

MEANS TO SOLVE THE PROBLEMS

In order to solve the above-mentioned problems, a first aspect of the present invention provides an exhaled breath visualization system comprising:

an infrared camera that detects light in the infrared region emitted from a face of a subject to obtain image data; a processing unit that subtracts, from first image data obtained at a first time, second image data obtained at a second time prior to the first time, to generate processed image data; and an output unit that outputs the processed image data. The first time is a certain time t1 that can be arbitrarily selected. The second time is an arbitrarily selectable time t2 prior to the first time (for example, t1 > t2 in the 24-hour clock).

In the exhaled breath visualization system of the present invention, it is preferable that the infrared camera detects light in the mid-infrared region.

Further, in the exhaled breath visualization system of the present invention, it is preferable that the processing unit uses, as the second image data, an average of image data captured over a predetermined period prior to the first time.

Further, a second aspect of the present invention provides an exhaled breath visualization method comprising:

a procedure for detecting light in the infrared region emitted from a face of a subject to obtain image data;
a procedure for subtracting, from first image data obtained at a first time, second image data obtained at a second time prior to the first time, to generate processed image data; and
a procedure for outputting the processed image data.

Further, a third aspect of the present invention provides an exhaled breath evaluation system comprising:

an infrared camera that detects light in the infrared region emitted from a face of a subject to obtain image data;
a processing unit that subtracts, from first image data obtained at a first time, second image data obtained at a second time prior to the first time, to generate processed image data;
a setting unit that sets a region of interest below the nostrils of the subject processed image data; and
an evaluation unit that calculates the flow velocity of the exhaled breath of the subject from the change over time in signal intensity on the region of interest.

In the exhaled breath evaluation system of the present invention, it is preferable that the evaluation unit calculates an opening area of the nostril from a diameter of the nostril, and calculates a flow rate of the exhaled breath by using the flow velocity and the opening area.

In the exhaled breath evaluation system of the present invention, it is preferable that the evaluation unit evaluates the energy metabolism of the subject from the flow rate of the exhaled breath by referring to data showing a relationship between energy metabolism and volume of the exhaled breath.

Further, a fourth aspect of the present invention provides an exhaled breath evaluation method comprising:

a procedure for detecting light in the infrared region emitted from a face of a subject to obtain image data;
a procedure for subtracting, from first image data obtained at a first time, second image data obtained at a second time prior to the first time, to generate processed image data;
a procedure for setting a region of interest below the nostrils of the subject processed image data; and
a procedure for calculating the flow velocity of the exhaled breath of the subject from the change over time in signal intensity on the region of interest.

EFFECT OF THE INVENTION

According to the present invention, the configuration is simple and the exhaled breath can be reliably visualized even when the front image of the face of the subject is captured by a camera. Therefore, it can be used in a variety of situations.

BRIEF EXPLANATION OF DRWAINGS

FIG. 1 is a schematic diagram of the system 1 and method according to the present embodiment.
FIG. 2 is a diagram showing an example of change in strength of the exhaled breath of a subject P over time.
FIG. 3 is a flowchart showing the procedures for visualizing exhaled breath.
FIG. 4 is a block diagram showing the functional configuration of the system 1.
FIG. 5 is a block diagram showing the hardware configuration of a computer 100 included in the system 1.
FIG. 6 is a flowchart showing the procedures for evaluating exhaled breath.
FIG. 7 is a flowchart showing the detailed procedures of the exhaled breath evaluation.
FIG. 8 is a diagram showing an example of an image formed (resliced) by setting a region of interest (ROI) and arranging the ROI along the time axis.
FIG. 9 is a diagram showing an example of a method for calculating a gradient from the resliced image.
FIG. 10 is a diagram showing another technique for setting an ROI, performing Reslice, and extracting a gradient.
FIG. 11 is a diagram showing an example of the procedures for calculating a volume of the exhaled breath.
FIG. 12 is a diagram showing an example of a method for extracting heart rate data from a temperature image of the face of the subject P.

MODE FOR CARRYING OUT THE INVENTION

In the following, by referring the drawings, the typical embodiments of the exhaled breath visualization system and method according to the present invention are explained in detail. However, the present invention is not limited to the drawings. Further, since these drawings are presented to explain the concept of the present invention, there are cases where sizes, ratios and numbers are exaggerated or simplified as necessary for ease of understanding.

First, the exhaled breath visualization system and the method thereof according to the present invention will be described, and then the exhaled breath evaluation system and the method thereof will be described. In the following description, the exhaled breath visualization system and the exhaled breath evaluation system will be collectively referred to as system 1.

### 1. Visualization of Exhaled Breath

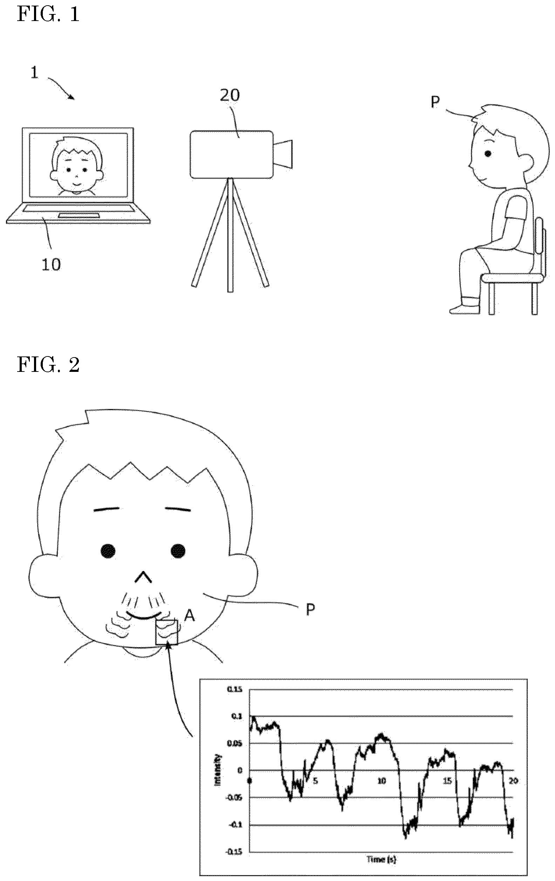

A method and system for visualizing the exhaled breath of a subject P will be described with reference to FIG. 1 to FIG. 5. The subject P is typically a patient, but is not limited thereto.

### 1-1. Exhaled Breath Visualization Method

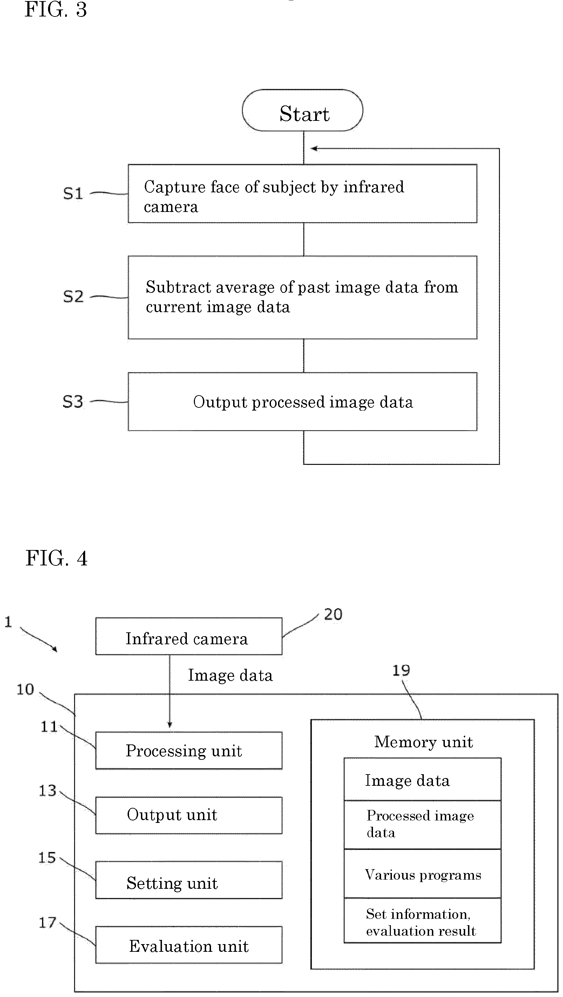

As shown in FIG. 3, the visualization of exhaled breath in this embodiment includes the procedures for (1) capturing images by using an infrared camera, (2) processing the image data, and (3) outputting the processed image data. Each procedure will be described in detail below.

### (1) Capturing with an infrared camera

As shown in FIG. 1, image data of the face of the subject P is captured via an infrared camera 20 (step S1 in FIG. 3). This embodiment has the feature that the exhaled breath can be reliably visualized even when the infrared camera 20 captures a front image of the face of the subject P, but the subject P may be facing diagonally or to the side with respect to the infrared camera 20.

The infrared camera 20 includes a detector that detects light in the infrared region (wavelengths of 0.7 $\mu$m to 1,000 $\mu$m), and this detector may be either a quantum type infrared detector or a thermal type infrared detector.

It is preferable that the infrared camera 20 detects light in the mid-infrared region, particularly light with wavelengths of 3 $\mu$m to 5 $\mu$m. Since the strong absorption band of $CO_2$ exists at 4.2 $\mu$m to 4.3 $\mu$m, the flow of $CO_2$ in the exhaled breath of the subject P can be reflected in the image data.

The frame rate of the infrared camera 20 is not particularly limited as long as the exhaled breath of the subject P can be adequately visually recognized, but for example, 30 fps and 60 fps can be used.

In this embodiment, it is assumed that the subject P is stationary (for example, sitting in a chair or lying down), but the subject P may also be moving (for example, moving or training by walking or running). Known tracking techniques are available for tracking the face of the subject P in motion.

### (2) Processing Image Data

The processed image data is generated by subtracting the second image data captured at the second time prior to the first time from the first image data (for instance, current image data) captured by the infrared camera 20 at the first time (step S2 in FIG. 3). The first time point is an arbitrarily selectable time t1, and may be, for example, the current time. At this time, the first image data can be called current image data. The second time is a time t2 that can be arbitrarily selected before the first time, that is, a time in the past. Therefore, for example, in the 24-hour clock, the relation of t1 > t2 can be established, and the second image data can be said to be past image data. It should be noted that some modification or correction may be applied to the first image data and the second image data prior to generating the processed image data.

In this process, an average of image data captured over a predetermined period of time in the past can be suitably used as the second image data. Here, the predetermined period of time may be, for example, 10 seconds to 50 seconds, and more preferably, 20 seconds to 45 seconds. However, if the respiration of the subject P is irregular, it is preferable to use a predetermined period of time of 20 seconds or more. For example, when the frame rate is 60 fps, 2400 pieces of image data are generated in 40 seconds.

The average of the past image data is calculated, for example, by adding up all pixel values for the predetermined period of time for each corresponding pixel and dividing the sum by the total number of images, but is not limited thereto.

The generation of the processed image data may be performed every time when the infrared camera generates image data, or may be performed at preset time intervals. In the latter case, one piece of the processed image data is generated every time when a predetermined number of pieces of image data are generated by the infrared camera.

The generation of the processed image data may be performed, for example, by using the free software "Imaged" (https://imagej.net/ij/index.html). Of course, other software having similar functions may be used.

(3) Output of Processed Image Data

The processed image data is output (step S3 in FIG. 3). Examples of the output form include displaying on a display such as a monitor, transmitting to an external display ·projector, and printing. The processed image data may be stored in a storage device.

The image shown in the upper left corner of FIG. 2 shows an example of the image data displayed on a computer monitor. By displaying these images in chronological order, the manner in which gas flows from the nostrils of the subject P can be visually grasped. For example, on the computer monitor, the movement of the exhaled breath of the subject P is displayed as the fluctuation of multiple thin linear lines, and can be understood as if it were a fluctuation of a white haze.

This is also shown in the graph at the bottom right corner of FIG. 2. That is, the signal intensity in the region A below the mouth of the subject P changes with a certain degree of regularity (i.e., periodically).

It goes without saying that the image exemplified in the upper left corner of FIG. 2 may be displayed on the display alone or may be accompanied by the graph display.

By using the exhaled breath visualization method according to this embodiment, it is possible to visualize the exhaled breath of the subject P facing the camera. In other words, even with the face of the subject P as a background, the exhaled breath of the subject P can be visually grasped.

1-2. Breath Visualization System

The system 1 which realizes the exhaled breath visualization method described above will be explained.

As shown in FIG. 4, the system 1 includes an infrared camera 20, a processing unit 11 and an output unit 13. The system 1 for realizing the exhaled breath visualization method does not necessarily include the setting unit 15 and the evaluation unit 17. The processing unit 11 and the output unit 13 can be realized as one function of the computer 10.

The infrared camera 20 was described in 1-1(1) above. In this embodiment, the infrared camera 20 can be a FLIR A6700sc available from Teledyne FLIR LLC.

The main features of the FLIR A6700sc are as follows:

Resolution: 640 x 512 pixels
Time resolution: 60 fps
Wavelength sensitivity: 3 to 5 $\mu$m
Dynamic range: 14 bit
Temperature resolution: 0.018°C

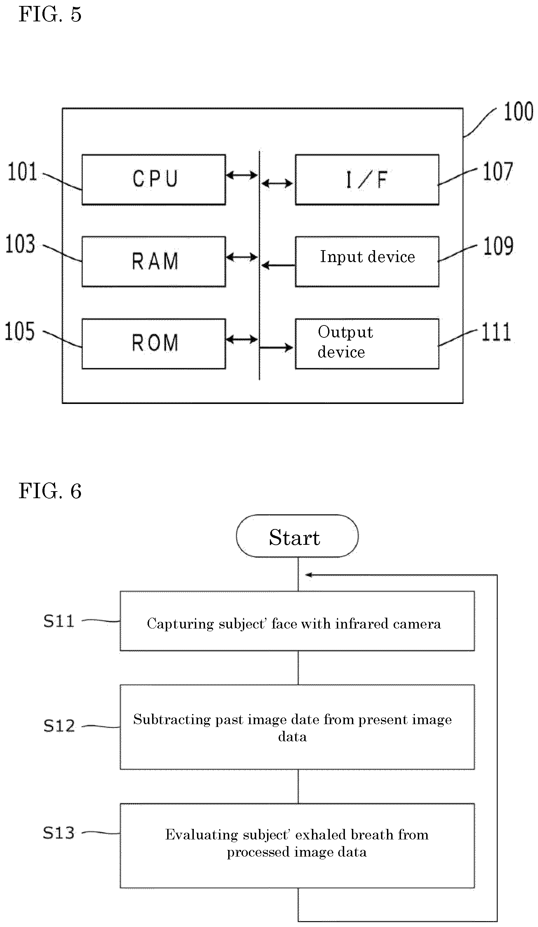

The processing unit 11 executes the above-mentioned procedure 1-1(2). In order to realize the processing unit 11, the computer 100 includes a CPU 101, a RAM 103, and a ROM 105 (see FIG. 5). The CPU 101 is a circuit that functions as a processing unit 11 by loading programs and various data stored in the ROM 105 into the RAM 103 and executing.

The output unit 13 executes the above-mentioned procedure 1-1(3). In order to realize the output section 13, the computer 100 includes a communication interface 107 and an output device 111. The output device 111 includes a computer display, a projector, a head mounted display, and a printer. Further, the communication interface 107 is used for communication with the external devices such as a computer display, a projector, a head mounted display, and a printer.

Note that, the communication interface 107 is also used to capture the image data from the infrared camera 20. Further, the input device 109 includes a mouse, a keyboard, and a touch panel. The infrared camera 20 may be included in the input device 109.

2. Assessment of Exhaled Breath

The method and system for evaluating the exhaled breath of the subject P will be described with reference to FIG. 4 to FIG. 11.

2-1. Breath Evaluation Method

As shown in FIG. 6, the exhaled breath evaluation method according to this embodiment includes the above-mentioned (1) capturing with the infrared camera and (2) processing of the image data (steps S11 and S12), as well as the evaluation of the exhaled breath (step S13). The exhaled breath evaluation method may or may not include the above-mentioned (3) outputting of the processed image data described above.

The evaluation of the exhaled breath includes (4) setting a region of interest, (5) calculating the speed and flow rate of the exhaled breath, and (6) evaluation. Procedures (4), (5) and (6) are explained in detail below.

(4) Setting the Region of Interest

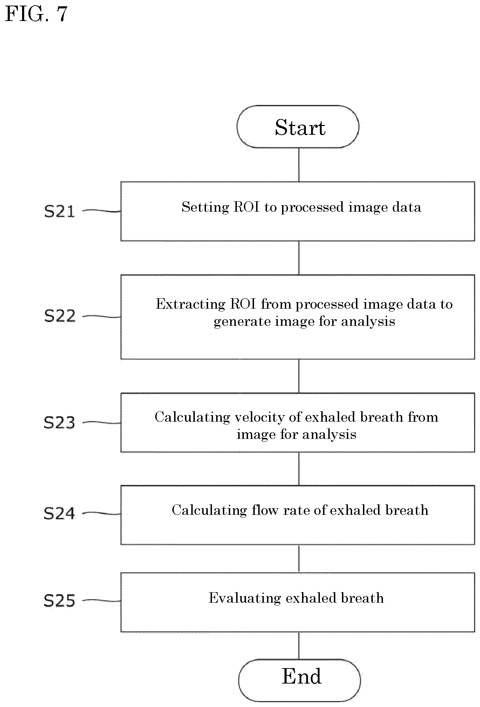

The region of interest (ROI) is set on the image data processed in the above-mentioned 1-1(2) (step S21 in FIG. 7). The ROI refers to a region of image data that is selected as a target for operation, and is also called a "target region" or "region of interest."

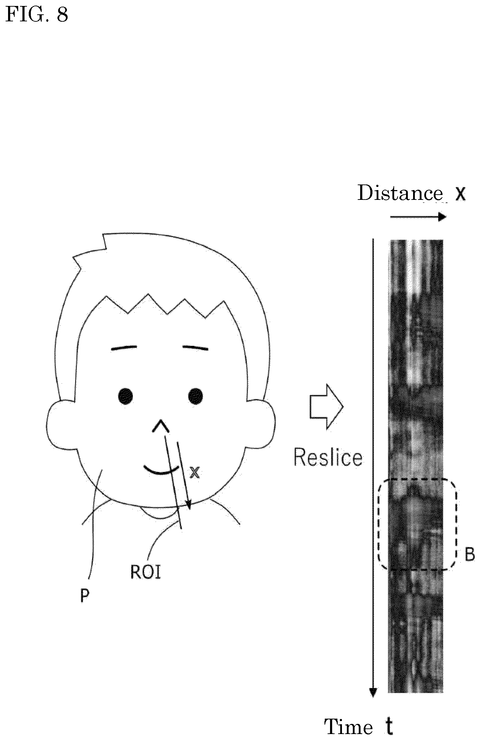

The ROI is set below the nostrils of the subject P. For example, as shown on the left side of FIG. 8, the ROI may be set in a linear or strip-shaped region extending downward from the nostrils of the subject P (i.e., on the flow path of the exhaled breath), but is not limited thereto.

The ROI may be set manually by the user, or may be set automatically based on the positions of the nose and mouth, the flow of exhaled breath, by using pattern recognition technology.

(5) Calculation of flow velocity and flow rate of exhaled breath

Next, the flow rate of the exhaled breath of the subject P is calculated based on the change in signal intensity on the ROI over time. The flow rate is calculated, for example, as follows.

That is, image for analysis is generated by extracting ROIs from a group of processed image data and arranging in chronological order (Reslice) (step S22 in FIG. 7). In the example of the image for analysis shown on the right side of FIG. 8, the horizontal axis indicates distance x along the ROI, and the vertical axis indicates time t. Therefore, the striped pattern seen in this figure indicates how the signal intensity on the ROI varies with time. The flow (flow velocity) of the exhaled breath can be grasped from this image for analysis (step S23 in FIG. 7).

Then, the angle θ and the length (distance) L of the striped bands are obtained from the image for analysis. Note that, the distance L may be appropriately corrected.

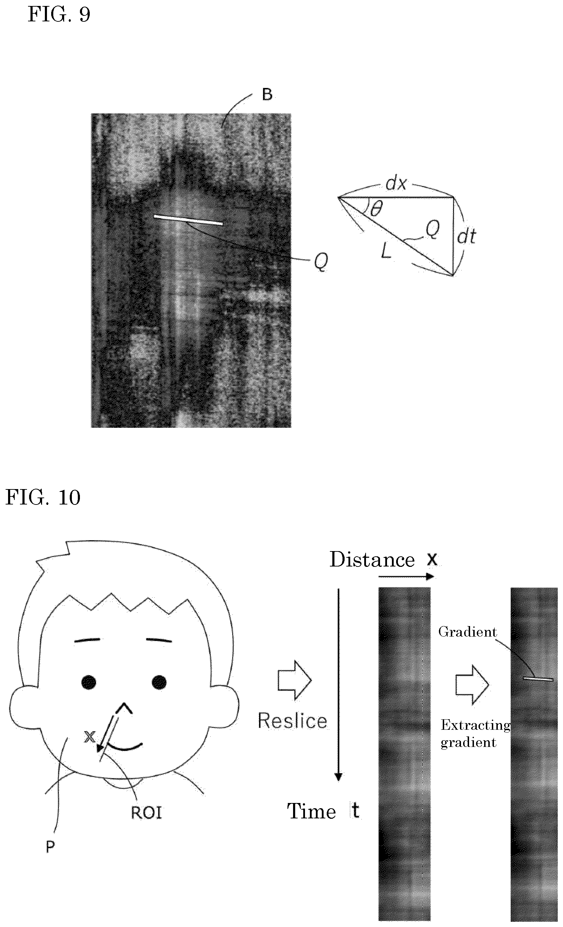

A calculation example of the angle θ and the distance L will be given. The image on the left side of FIG. 9 is an enlarged image of region B in FIG. 8. Focusing on the striped bands in this image, a line Q is drawn. Then, the length L of this line and the angle θ with the horizontal axis are calculated.

As shown on the right side of FIG. 9, there is a known relationship between the distance L, the angle θ, the displacement dx along the x-axis, and the displacement dt along the time axis. Using this relationship, dx = L cos θ and dt = L sin θ are calculated from the calculated angle θ and distance L, and furthermore, the gradient of the band (line Q), that is, the velocity of the exhaled breath v = dx / dt, is calculated from the calculated dx and dt.

More specifically, the velocity v can be calculated as follows, for example.

That is, the order of x, L, is the length, and t is the time.

However, in the case of the image processing, since x and L are given as the distance between pixels (number of pixels), it is necessary to correct the number of pixels to distance by using the relationship between the number of pixels and distance (mm/pixel). Further, t is a time period that is determined by the frame rate. For example, at 60 fps, it is 0.0167 sec/pixel, and this is used for correction.

The line Q is drawn by using the software for image processing, and the distance L and angle θ of the line Q are determined by analyzing this line.

Accordingly, dx and dt are calculated as follows:

$$\text{dx (mm)} = \text{L cos }\theta\text{ (pixel)} \times \text{distance correction coefficient (mm/pixel)}$$

$$\text{dt (sec)} = \text{L sin }\theta\text{ (pixel)} \times \text{time correction coefficient (sec/pixel)}$$

As a result, the velocity v is obtained by using the following equation.

$$\text{v (mm/sec)} = \text{dx (mm)} / \text{dt (sec)}$$

Alternatively, L can be treated as a length rather than pixels. In this case, it is necessary to use dx as it is and convert dt from a number of pixels to a time.

Accordingly, by knowing the velocity v of the exhaled breath, it is possible to determine whether the subject P is breathing. Further, the breathing rhythm of the subject P can be known from the velocity v of the exhaled breath (see, for example, the graph on the lower right of FIG. 2).

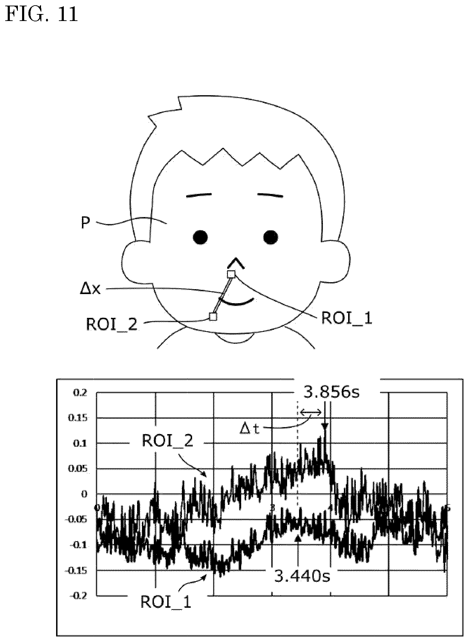

A more specific processing example is shown in FIG. 10 and FIG. 11. However, the present invention is not limited to the following processing example.

In the image data shown on the left side of FIG. 10, the ROI (straight line) is placed on the flow path of the exhaled breath ($CO_2$) from the nostrils of the subject P. Next, a portion corresponding to the ROI is extracted from the group of image data, and the extracted elements are arranged in chronological order to generate an image for analysis (see the central image in FIG. 10). This image for analysis may be denoised by using a smoothing filter such as a Gaussian filter. Further, the contrast of the image may be enhanced by contrast conversion such as Enhanced Contrast.

Then, an edge detection method such as Canny edge detection is used to extract the gradient of the striped pattern in the image. Furthermore, a line detection method such as Ridge detection (Hough transform) is used to set the ROI at the gradient of the striped pattern. An approximation of the gradient is obtained from the XY coordinates of the ROI (see the image on the right side of FIG. 10).

Alternatively, in the image data shown in the upper part of FIG. 11, two ROIs are set on the flow path of the exhaled breath ($CO_2$) from the nostrils of the subject P. In the figure, the upstream ROI is indicated as ROI_1, and the downstream ROI is indicated as ROI_2. Next, the distance $\Delta x$ between these ROIs is calculated. Then, the time difference $\Delta t$ between the peaks in the graph showing the $CO_2$ fluctuations in each ROI (for example, the lower graph in FIG. 11) is read. In the figure, the peak of ROI_1 arrives at 3.440 seconds, while the peak of ROI_2 arrives at 3.856 seconds, resulting in a time difference $\Delta t = 0.416$. This makes it possible to calculate the flow velocity of the exhaled breath $v = \Delta x / \Delta t$.

The flow rate F of the exhaled breath is calculated by using the calculated velocity v of the exhaled breath (step S24 in FIG. 7).

That is, the nostril opening area S of the subject P is obtained from the processed image data, and the flow rate F of the exhaled breath is calculated by using the opening area S and the velocity v of the exhaled breath. For example, the nostrils of subject P are approximated as circles, the opening area $S = D^2\pi/4$ is calculated from the diameter D of the nostrils, and the flow rate F of the exhaled breath is calculated according to $F = S \times V$.

(6) Evaluation

Next, the exhaled breath of the subject P is evaluated by using the obtained velocity v of the exhaled breath and the flow rate F of the exhaled breath (step S25 in Figure 7). An example of the evaluation of the exhaled breath is the evaluation of energy metabolism, in addition to the presence or absence and rhythm of breathing mentioned above.

In the evaluation of the energy metabolism, referring the data showing the relationship between the energy metabolism and the exhaled breath volume, the energy metabolism of the subject P is estimated from the flow rate of exhaled breath. Publicly available data can be used to indicate the relationship between the energy metabolism and the exhaled breath volume. For example, "$CO_2$ amount of human exhalation (adult male)" according to the Japanese Industrial Standard (JIS A 1406:1974) and "$CO_2$ amount of exhalation by work intensity" according to the Heating, Air-Conditioning and Sanitary Engineers of Japan Standard (HASS 102-1996) can be used. Such data may be stored in the computer 10.

2-2. Breath Evaluation System

The system 1 that realizes the exhaled breath evaluation method described above will be explained.

As shown in FIG. 4, the system 1 further includes a setting unit 15 and an evaluation unit 17 in addition to the infrared camera 20 and the processing unit 11 described above. The system 1 for realizing the exhaled breath evaluation method does not necessarily include the output unit 13.

Since the infrared camera 20 and the processing unit 11 have been explained above, explanation thereof will be omitted here.

The setting unit 15 can execute the procedure described in 2-1(4) above. The evaluation unit 17 can execute the procedure described in 2-1(5) above. The setting unit 15 and the evaluation unit 17 can be executed as one function of the computer 10 described above. That is, the CPU 101 functions as the setting unit 15 and the evaluation unit 17 by loading the programs and various data stored in the ROM 105 into the RAM 103 and executing them (see FIG. 5). Note that, the processing unit 11, the output unit 13, the setting unit 15, and the evaluation unit 17 may be executed by one computer or by multiple computers.

3. Measuring Heart Rate and Body Temperature

In this embodiment, in addition to measuring the exhaled breath of the subject P, the heart rate and the body temperature of the subject P may also be measured. Outline of the measurements of the heart rate and the body temperature will be explained below.

3-1. Heart Rate Measurement

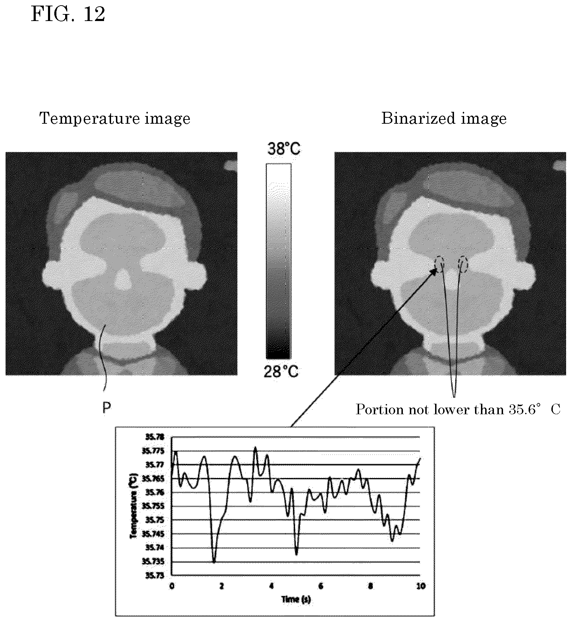

The face of the subject P is captured with the infrared camera 20, and a temperature image showing the temperature distribution on the face of the subject P is obtained (see the image on the top left of FIG. 12). That is, the infrared camera 20 is used as a thermograph. This temperature image is subjected to image processing to generate a binarized image, and from the generated image, portions having a predetermined temperature (for example, 35.6° C) or higher are extracted (see the image on the upper right of FIG. 12). The graph shown on the bottom of FIG. 12 shows an example of the change in temperature over time in the extracted portion.

A filter such as a Bandpass Butterworth filter (0.8 to 1.5 Hz) is applied to the data indicating the change in temperature over time in the extracted portion to add a band restriction to the data. Then, frequency analysis (Fourier transform) is applied to the band-limited data to obtain heart rate data.

Therefore, the setting unit 15 sets a predetermined portion (for example, the inner corner of the eye) in the temperature image data obtained from the infrared camera 20 as the ROI. Each time the evaluation unit 15 obtains temperature image data, it can obtain heart rate data by extracting the temperature data from this portion and performing frequency analysis.

3-2. Body Temperature Measurement

Body temperature data of the subject P can also be obtained by capturing the face of the subject P with the infrared camera 20. For example, the temperature of the inner corner of the eye of the subject P can be measured by using the infrared camera 20 and used as a representative value of body temperature. According to one study, the skin of the inner corner of the human eye is less susceptible to external factors such as the outside air, while the skin on other parts of the head is more susceptible to external factors. For example, when standing outside during cold weather, the temperature of the forehead drops significantly compared to the temperature inside the body, but no such dramatic change occurs at the inner corner of the eye.

Therefore, for example, the setting unit 15 sets a predetermined portion (for example, the inner corner of the eye) in the temperature image data obtained from the infrared camera 20 as the ROI. Each time the evaluation unit 15 obtains temperature image data, the temperature data may be extracted from this portion.

4. Effects of this embodiment

In this embodiment, the infrared camera 20 is used that is sensitive to the absorption band of $CO_2$ contained in human exhaled breath. Further, image data for display is generated by subtracting the past photographic data from the real-time or latest photographic data. This makes it possible to visualize the exhaled breath of the subject P who faces the infrared camera 20. In other words, there is no need to face the camera sideways or to prepare multiple types of cameras in order to distinguish the temperature of the exhaled breath of the subject P from the background, as in the conventional method.

Therefore, for example, by visualizing the exhaled breath of the patient through a monitor, a doctor can monitor the breathing of the patient without coming into contact with the patient. Accordingly, this technology is expected to be applied to remote medical care as well as screening for people infected with infectious diseases such as COVID-19.

In addition, it is possible to evaluate the breathing of not only people who are stationary relative to the camera, but also people who are sleeping, moving, or training. Therefore, the present technology can be widely used in, for example, evaluating breathing disorders during sleep (for example, sleep apnea syndrome), evaluating the effectiveness of training, determining changes in physical condition, monitoring, watching systems, emergency notification systems, and the like.

In addition, in this embodiment, the visualized exhaled breath can be analyzed to evaluate the exhaled breath volume. This makes it possible to evaluate the energy metabolic rate of the subject P in the non-contact manner without using the conventional exhaled breath gas analyzer (for example, a human calorimeter, a Douglas bag, an indirect calorimeter). Therefore, the present technology can be used for routine health monitoring and management of humans.

Furthermore, by analyzing the specific portions of the subject P captured by the infrared camera 20, the body temperature (core body temperature) and heart rate of the subject P can be evaluated. In other words, a single infrared camera 20 can visualize breathing, and evaluate the energy metabolic rate, core body temperature, and heart rate all at once. Therefore, it is possible to reduce the number of components in the system and suppress manufacturing costs.

In addition to the human exhaled breath, this technology can be used to detect $CO_2$ leaks in $CO_2$ firefighting equipment, $CO_2$ refrigerant air conditioning equipment, water heater, combustion appliance (stoves, etc.), $CO_2$ storage facilities, and the like.

Representative embodiments of the present invention have been described above, but the present invention is not limited thereto, and various design modifications are possible, which are also included in the present invention.

For example, when obtaining the heart rate, a filter can be applied to the data showing the change in temperature over time, and peak detection can be performed on the processed data to determine the peak-to-peak time. Alternatively, the heart rate (bpm) can also be calculated as 60 / (peak-to-peak time).

Since there are multiple infrared $CO_2$ absorption bands, it is possible to visualize exhaled breath in a manner similar to that of this embodiment by using, for example, a far-infrared camera (sensitive to 8 to 14 $\mu$m).

For the temperature measurement, machine learning may be used for facial landmark detection. For example, the machine learning library dlib (http://dlib.net/) provides tools that can be used to detect the inner corner of the eye (for example, Number 40 and Number 43). Therefore, it is possible to measure the body temperature at the same location while tracking the inner corner of the eye.

One aspect of evaluation of the exhaled breath can include the evaluation of respiratory rate. For example, the respiration rate can be calculated by applying a filter with a band pass of 0.1 to 0.4 Hz to the graph on the lower right of FIG. 2 and performing a Fourier transform on the filtered data. Alternatively, the respiration rate can be obtained by performing the peak detection and the peak-to-peak processing on the graph shown in FIG.2.

Alternatively, the temperature image of the face of the subject P captured by the infrared camera 20 can be used to detect the vascular abnormalities such as carotid artery stenosis, arteriosclerosis, and high blood pressure. That is, since the temperature image contains information about the facial artery that branches off from the carotid artery, it is expected that the vascular abnormalities can be detected from, for example, the change in temperature distribution near the eye socket.

Specifically, a threshold is set for the temperature image of the face of the subject P. High temperature areas exceeding this threshold (for example, the suborbital, supraorbital, and nasolabial fold areas) are selected, and the total area of the areas, the left-right difference or left-right ratio, and the left-right difference in average temperature are measured. Here, the total area can be used as an index for the high blood pressure or arteriosclerosis, and the left-right difference or left-right ratio of the area and the average temperature can be used as an index for carotid artery stenosis, respectively. The area may be substituted with the number of pixels belonging to the corresponding area.

For example, in the temperature image of the subject P, the total area of the high temperature areas around both eyes can be calculated and the areas of the left and right areas can be compared, or the average temperatures of the left and right areas can be compared. These processes can be performed, for example, by the above-mentioned free software ImageJ.

Note that, there are several papers suggesting the relationship between the facial temperature and the carotid artery stenosis. It has been pointed out the possibility that carotid artery stenosis may change arterial blood flow, which may in turn change the temperature distribution along the arterial path running through the face. From this reason, when no substantial difference is observed between the left and right sides, the subject P may be judged to be healthy, or, when an imbalance between the left and right sides is observed, it may be judged that the subject P may have unilateral stenosis.

Such anomaly detection techniques can be incorporated into the system 1, thereby enabling the system 1 to evaluate various health conditions of the subject P.

EXPLANATION OF SYMBOLS

1 System

10 Computer

11 Processing unit

13 Output unit

15 Setting unit

17 Evaluation unit

19 Memory unit

20 Infrared camera

**Claims**

1.  An exhaled breath visualization system comprising:

    an infrared camera that detects light in the infrared region emitted from a face of a subject to obtain image data;
    a processing unit that subtracts, from first image data obtained at a first time, second image data obtained at a second time prior to the first time, to generate processed image data; and
    an output unit that outputs the processed image data.

2.  The exhaled breath visualization system according to claim 1, wherein the infrared camera detects light in a mid-infrared region.

3.  The exhaled breath visualization system according to claim 1 or 2,
    wherein the processing unit uses, as the second image data, an average of image data captured over a predetermined period of time before the first time.

4.  An exhaled breath visualization method comprising:

    a procedure for detecting light in the infrared region emitted from a face of a subject to obtain image data;
    a procedure for subtracting, from first image data obtained at a first time, second image data obtained at a second time prior to the first time, to generate processed image data; and
    a procedure for outputting the processed image data.

5.  An exhaled breath evaluation system comprising:

    an infrared camera that detects light in the infrared region emitted from a face of a subject to obtain image data;
    a processing unit that subtracts, from first image data obtained at a first time, second image data obtained at a second time prior to the first time, to generate processed image data;
    a setting unit that sets a region of interest below the nostrils of the subject processed image data; and
    an evaluation unit that calculates the flow velocity of the exhaled breath of the subject from the change over time in signal intensity on the region of interest.

6.  The exhaled breath evaluation system according to claim 5, wherein the evaluation unit calculates an opening area of the nostril from a diameter of the nostril, and calculates a flow rate of the exhaled breath by using the flow velocity and the opening area.

7.  The exhaled breath evaluation system according to claim 6, wherein the evaluation unit evaluates the energy metabolism of the subject from the flow rate of the exhaled breath by referring to data showing a relationship between energy metabolism and volume of the exhaled breath.

8.  An exhaled breath evaluation method comprising:

    a procedure for detecting light in the infrared region emitted from a face of a subject to obtain image data;
    a procedure for subtracting, from first image data obtained at a first time, second image data obtained at a second time prior to the first time, to generate processed image data;
    a procedure for setting a region of interest below the nostrils of the subject processed image data; and
    a procedure for calculating the flow velocity of the exhaled breath of the subject from the change over time in signal intensity on the region of interest.

FIG. 1

FIG. 2

FIG. 3

```
                    ┌──────────────┐
                    │    Start     │◄─────────────┐
                    └──────┬───────┘              │
                           │                      │
         ┌─────────────────▼─────────────────┐    │
  S1 ────┤ Capture face of subject by infrared│    │
         │ camera                             │    │
         └─────────────────┬─────────────────┘    │
                           │                      │
         ┌─────────────────▼─────────────────┐    │
  S2 ────┤ Subtract average of past image data│    │
         │ from current image data            │    │
         └─────────────────┬─────────────────┘    │
                           │                      │
         ┌─────────────────▼─────────────────┐    │
  S3 ────┤    Output processed image data     │    │
         └─────────────────┬─────────────────┘    │
                           │                      │
                           └──────────────────────┘
```

FIG. 4

FIG. 5

100
101 — CPU ←→ ←→ I / F — 107

103 — RAM ←→ ← Input device — 109

105 — ROM ←→ → Output device — 111

FIG. 6

( Start )

S11 — Capturing subject' face with infrared camera

S12 — Subtracting past image date from present image data

S13 — Evaluating subject' exhaled breath from processed image data

FIG. 7

```
                    ╭─────────────╮
                    │    Start     │
                    ╰──────┬──────╯
                           │
  S21 ──────┌──────────────┴───────────────┐
            │  Setting ROI to processed image data  │
            └──────────────┬───────────────┘
                           │
  S22 ──────┌──────────────┴───────────────┐
            │  Extracting ROI from processed image data to │
            │       generate image for analysis      │
            └──────────────┬───────────────┘
                           │
  S23 ──────┌──────────────┴───────────────┐
            │  Calculating velocity of exhaled breath from │
            │          image for analysis          │
            └──────────────┬───────────────┘
                           │
  S24 ──────┌──────────────┴───────────────┐
            │  Calculating flow rate of exhaled breath │
            └──────────────┬───────────────┘
                           │
  S25 ──────┌──────────────┴───────────────┐
            │      Evaluating exhaled breath       │
            └──────────────┬───────────────┘
                           │
                    ╭──────┴──────╮
                    │     End      │
                    ╰─────────────╯
```

FIG. 8

Distance X

Reslice

Time t

FIG. 9

FIG. 10

FIG. 11

FIG. 12

Temperature image                                    Binarized image

38°C

28°C

P

Portion not lower than 35.6° C

## INTERNATIONAL SEARCH REPORT

| International application No. |
|---|
| **PCT/JP2023/017426** |

### A. CLASSIFICATION OF SUBJECT MATTER

*A61B 5/08*(2006.01)i; *A61B 5/00*(2006.01)i
FI: A61B5/08; A61B5/00 B

According to International Patent Classification (IPC) or to both national classification and IPC

### B. FIELDS SEARCHED

Minimum documentation searched (classification system followed by classification symbols)

A61B5/00-5/398

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | US 2022/0047160 A1 (RESEARCH & BUSINESS FOUNDATION SUNGKYUNKWAN UNIVERSITY) 17 February 2022 (2022-02-17) paragraphs [0105], [0194]-[0197], [0222], fig. 7 | 1-2, 4 |
| A | | 3, 5-8 |
| X | JP 2008-086741 A (TOMONO, Akira) 17 April 2008 (2008-04-17) paragraphs [0150], [0156]-[0159], fig. 5, 6 | 1-2, 4 |
| A | | 3, 5-8 |
| A | CN 114157807 A (JIANGSU MACRO-INTELLIGENT MEDICAL TECHNOLOGY CO., LTD) 08 March 2022 (2022-03-08) paragraphs [0026], [0031], [0042] | 1-8 |
| A | CN 113887474 A (SHENZHEN SENSETIME TECHNOLOGY CO., LTD.) 04 January 2022 (2022-01-04) entire text, all drawings | 1-8 |

☑ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **29 June 2023** | **11 July 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/017426**

**C.     DOCUMENTS CONSIDERED TO BE RELEVANT**

| Category* | Citation of document, with indication, where appropriate, of the relevant passages | Relevant to claim No. |
|---|---|---|
| A | 塙大ほか. ホームヘルスケアシステムにおける遠赤外線画像を用いた鼻呼吸検出法の提案. 電子情報通信学会論文誌. 2011, vol. J94-D, no. 1, pp. 260-263, ISSN: 1880-4535, (HANAWA, Dai et al. A proposal of nasal breathing detection method by using far infrared imaging in a home health care system. IEICE Transactions on Information and Systems.)<br>　　entire text | 1-8 |
| A | HANAWA, Dai et al. Relationship between the Nasal Region on Facial Thermal Images and the Flow Velocity of Nasal Breathing. 2022 IEEE 4th Global Conference on Life Sciences and Technologies (LifeTech). March 2022, pp. 30-31, DOI: 10.1109/LifeTech53646.2022.9754745<br>　　entire text | 1-8 |

Form PCT/ISA/210 (second sheet) (January 2015)

20

INTERNATIONAL SEARCH REPORT
Information on patent family members

International application No.

**PCT/JP2023/017426**

| Patent document cited in search report | | | Publication date (day/month/year) | Patent family member(s) | | | Publication date (day/month/year) |
|---|---|---|---|---|---|---|---|
| US | 2022/0047160 | A1 | 17 February 2022 | KR | 10-2199020 | B1 | |
| JP | 2008-086741 | A | 17 April 2008 | (Family: none) | | | |
| CN | 114157807 | A | 08 March 2022 | (Family: none) | | | |
| CN | 113887474 | A | 04 January 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. SUN et al.** Remote sensing of multiple vital signs using a CMOS camera-equipped infrared thermography system and its clinical application in rapidly screening patients with suspected infectious diseases. *International Journal of Infectious Diseases*, 2017, vol. 55, 113-117

- **J. FEI et al.** Imaging Breathing Rate in the CO2 Absorption Band. *Proceedings of the 2005 IEEE*, 700-705